# EUROPEAN PATENT APPLICATION

(11) **EP 4 209 244 A1**
(43) Date of publication of application: **12.07.2023**
(21) Application number: 21864454.0
(22) Date of filing: 06.09.2021
(51) Int. Cl.: A61M 25/00

(54) **CATHETER**

(30) Priority: 04.09.2020 JP 2020148824
(71) Applicant: Goodman Co., Ltd., Aichi 460-0008 (JP)
(72) Inventor: CHIZUKI, Toshihiko, Seto-shi, Aichi 489-0976 (JP); HIRATSUKA, Shinsuke, Seto-shi, Aichi 489-0976 (JP); TAMURA, Mizuki, Seto-shi, Aichi 489-0976 (JP); YOKOI, Tokun, Seto-shi, Aichi 489-0976 (JP)
(74) Representative: Epping - Hermann - Fischer
(86) International application number: PCT/JP2021/032574
(87) International publication number: WO 2022/050403

(57) **Abstract**

A catheter (1) includes a cylindrical reinforcement member (2), formed from a braided body obtained by weaving a plurality of first wires (3) and a plurality of second wires (4). A melting point of the plurality of first wires (3) is 500°C or more than a melting point of the plurality of second wires (4). At at least one end of both of end portions, bonding portions (5) are formed on some of a plurality of intersection points (Qc). The bonding portion (5) bonds a first wire (3[n]) and a second wire (4[n]) intersecting with each other at the intersection points (Qc[n]). The bonding portion (Qc[n]) bonds the first wire (3[n]) and the second wire (4[n]) by alloying the first wire (3[n]) and the second wire (4[n]) at a contact portion.

## Description

### Technical Field

The present disclosure relates to a catheter.

### Background art

With a catheter, it is necessary to cause a distal end portion to correctly reach a portion that is a target of treatment, in a blood vessel, and thus, a favorable pushing in performance is demanded. Thus, various types of catheter are proposed that include a reinforcement structure in order to realize this type of performance. Patent Literature 1 discloses a catheter including, as a reinforcement structure, a metal mesh formed from wires of stainless steel (SUS). In this catheter, intersection portions of a first group of wires and a second group of wires of the metal mesh are welded by being irradiated with laser light. Next, the laser light is irradiated onto the metal proximal mesh along a cutting line, and the metal mesh is cut. According to this processing method, end portions of the metal mesh do not easily come apart. Note that when welding using the irradiation of the laser light, the first group of wires and the second group of wires preferably fuse and become alloyed at respective contact portions therebetween, in order to maintain the strength of the welded portions.

### Citation List

### Patent Literature

Patent Literature 1: JP-2005-230318 A

### Summary of Invention

In order to increase its strength as the reinforcement structure, there is a case in which a metal mesh formed from a plurality of wires of different materials is used. A case will be described using an example in which the laser light is irradiated on the basis of the above-described processing method, on each of wires of two types of material for which a difference in respective melting points is large. When the laser light is irradiated at an intensity necessary to fuse the wire having the relatively high melting point (hereinafter referred to as a first wire) of the two types of wire, there is a possibility that wire that has the relatively low melting point (hereinafter referred to as a second wire) of the two types of wire, may melt, and that the second wire may be thermally cut. On the other hand, when the laser light is irradiated at an intensity necessary to fuse the second wire, there is a possibility that the first wire is not fused. In other words, in either of these cases, both the first wire and the second wire are not fused and do not become alloyed at the contact portion, and thus, there is a problem that there is a case in which the strength of the contact portion cannot be maintained.

An object of the present disclosure is to provide a catheter capable of making it difficult for end portions of a metal mesh to come apart, even when a plurality of wires having different melting points are used.

A catheter according to a first aspect of the present disclosure includes a cylindrical reinforcement member extending in an extension direction, formed from a braided body obtained by weaving, into a mesh shape, a plurality of first wires extending in a first direction and a plurality of second wires extending in a second direction intersecting the first direction. A melting point of the plurality of first wires is 500°C or more than a melting point of the plurality of second wires. At at least one end, of both of end portions in the extension direction of the reinforcement member, bonding portions are formed on some of a plurality of intersection points of intersection between the plurality of first wires and the plurality of second wires. The bonding portion bonds a first intersecting wire and a second intersecting wire, the first intersecting wire and the second intersecting wire being the plurality of first wires and the plurality of second wires intersecting with each other at the some of the plurality of intersection points. The bonding portion bonds the first intersecting wire and the second intersecting wire by alloying the first intersecting wire and the second intersecting wire at a contact portion between each of the first intersecting wire and the second intersecting wire.

In the catheter, the bonding portion is formed as a result of the first intersecting wire and the second intersecting wire being alloyed at the contact portion between each of the first intersecting wire and the second intersecting wire, and the first intersecting wire and the second intersecting wire being bonded. Thus, the catheter can make it difficult for the end portions in the extension direction of the reinforcement member to come apart, even when using, as the reinforcement member, the braided body that is formed from the plurality of first wires and the plurality of second wires having the different melting points.

According to a first aspect of the present disclosure, of the plurality of first wires and the plurality of second wires, respectively, at least one end, of both end portions thereof in the extension direction, may include a curved portion curving with respect to the extension direction. Even when each of the plurality of first wires and the plurality of second wires is curved at the curved portion, the catheter can maintain a state in which each of the plurality of first wires and the plurality of second wires are bonded together, as a result of alloying each of the plurality of first wires and the plurality of second wires at the bonding portion.

According to a first aspect of the present disclosure, of the second intersecting wire, a second distal end portion further to a distal end side than the bonding portion may be wound onto the first intersecting wire. In this case, the catheter can even more firmly bond the first intersecting wire and the second intersecting wire.

According to a first aspect of the present disclosure, the catheter may further include an inner layer tube having a cylindrical shape centered on a central axis of the reinforcement member, and disposed in a lumen of the reinforcement member, and an outer layer tube having a cylindrical shape centered on the central axis, and covering the reinforcement member from an outer side. When a wire or the like passes through the lumen of the reinforcement member, the inner layer tube can suppress the wire from becoming caught up on the reinforcement member. Further, the outer layer tube can suppress the reinforcement member from being exposed. Thus, the catheter can reduce a possibility of the reinforcement member becoming caught up on a blood vessel when the catheter passes through the blood vessel.

According to a first aspect of the present disclosure, a light transmittance of the inner layer tube may be higher than a light transmittance of the outer layer tube. When laser light is irradiated in order to bond the plurality of first wires and the plurality of second wires by heating the plurality of first wires and the plurality of second wires, the inner layer tube can suppress heat generation resulting from absorption of the laser light. Thus, it is possible to reduce a possibility of the inner layer tube melting or becoming damaged due to heat generation.

According to a first aspect of the present disclosure, a material of the inner layer tube may be polytetrafluoroethylene. In this case, the inner layer tube having the high light transmittance can be easily realized.

According to a first aspect of the present disclosure, the first intersecting wire may be disposed to one side, in a third direction, with respect to the second intersecting wire, the third direction being orthogonal to the first direction and the second direction. In this case, the first intersecting wire and the second intersecting wire can be easily bonded by irradiating the laser light from the one side in the third direction. Further, when the laser light is irradiated and the bonding portion is formed, more of the energy of the laser light is imparted to the first wire having the high melting point, and the first wire can be melted. Further, the imparting of the energy of the laser light to the second wire having the low melting point can be suppressed, and the second wire can be melted and thermally cut.

According to a first aspect of the present disclosure, a cross-sectional shape of one of the plurality of first wires and the plurality of second wires may be a circular shape, and the cross-sectional shape of the other of the plurality of first wires and the plurality of second wires may be a rectangular shape. Also, according to a first aspect of the present disclosure, the cross-sectional shape of the plurality of first wires may be the circular shape, and the cross-sectional shape of the plurality of second wires may be the rectangular shape. In this case, for the catheter, a process can be easily realized to form the bonding portion by alloying the first intersecting wire and the second intersecting wire at the contact portion between each of the first intersecting wire and the second intersecting wire.

According to a first aspect of the present disclosure, an absorption coefficient of the plurality of first wires may be greater than an absorption coefficient of the plurality of second wires. In this case, even when the bonding of the first wire and the second wire is realized by the one-time irradiation of the laser light, the first wire and the second wire can be appropriately melted and alloyed, and each can be bonded.

According to a first aspect of the present disclosure, of the second intersecting wire, a composition of a portion other than the bonding portion may be the same, regardless of a distance from the bonding portion. In this case, since the composition of the second wire can be stably maintained, the catheter can maintain the strength of the braided body.

According to a first aspect of the present disclosure, a material of the plurality of first wires may be tungsten, and a material of the plurality of second wires may be stainless steel. In this case, with the catheter, a favorable pushing in performance can be realized by the braided body. Further, since tungsten has superior shielding properties for radiation, verifying a position of the catheter inside a body can be easily performed using radiation exposure.

A manufacturing method of a catheter according to a second aspect of the present disclosure is a manufacturing method for manufacturing a catheter including
a cylindrical reinforcement member extending in an extension direction, formed from a braided body obtained by weaving, into a mesh shape, a plurality of first wires extending in a first direction and a plurality of second wires extending in a second direction intersecting the first direction, a melting point of the plurality of first wires being 500°C or more than a melting point of the plurality of second wires, at at least one end, of both of end portions in the extension direction of the reinforcement member, bonding portions being formed on some of a plurality of intersection points of intersection between the plurality of first wires and the plurality of second wires, the bonding portion bonding, by alloying at respective contact portions thereof, a first wire [n] and a second wire [n] intersecting each other at a bonding intersection point [n], of the plurality of intersection points, and the first wire [n] being disposed to one side, in a third direction, with respect to the second wire [n], the third direction being orthogonal to the first direction and the second direction at the bonding intersection point [n]. The manufacturing method includes:
a first irradiation step of alloying and bonding the first wire [n] and the second wire [n] at the bonding intersection point [n] and thermally cutting a section, of the second wire [n], further to one side in the second direction than the bonding intersection point [n], by irradiating, at the bonding intersection point [n], laser light from the one side in the third direction onto a first irradiation region [n] including at least a portion on the one side in the second direction of a bonding region [n] overlapping the first wire [n] and the second wire [n] when the braided body is viewed from the one side in the third direction, and a portion of the second wire [n] not overlapping the first wire [n], and
a second irradiation step of, after irradiating the laser light in the first irradiation step, cutting the first wire [n] at a position of a second irradiation region [n], by irradiating laser light from the one side in the third direction onto the second irradiation region [n] separated to one side in the first direction from the bonding intersection point [n] of the first wire [n].

The laser light irradiated in the first irradiation step first heats and melts a portion of the first wire [n], and next, at the same time as heating and melting, and thermally cutting the second wire [n], bonds the melted first wire [n] and second wire [n] by alloying the first wire [n] and the second wire [n]. The laser light irradiated in the second irradiation step heats and thermally cuts the first wire [n]. In this way, at least one side in the extension direction of the reinforcement member is unlikely to come apart, due to the bonding portion.

In the above-described manufacturing method, the bonding by alloying of the first wire [n] and the second wire [n], and the thermal cutting of the second wire [n] are simultaneously performed by the one-time irradiation of the laser light in the first irradiation step. Thus, it is possible to reduce a possibility of a deterioration in the precision of a positional relationship between a position of the bonding portion and a thermal cutting position of the second wire. Further, energy of the laser light is first imparted to the first wire [n], and the remaining energy is imparted to the second wire [n]. Thus, even when the melting point of the first wire is 500°C or more than the melting point of the second wire, the bonding of the first wire [n] and the second wire [n], and the thermal cutting of the first wire [n] can be efficiently performed by the one-time irradiation of the laser light.

In the second aspect, at least one of the first irradiation step and the second irradiation step may irradiate the laser light while changing, over time, energy imparted to the braided body by the irradiation of the laser light.

In this case, since it is possible to suppress heat from being transmitted toward the vicinity of an irradiation region of the laser light in the first wire and the second wire, it is possible to prevent the occurrence of melting, vaporization, and extinction of the first wire and the second wire in the vicinity of the irradiation region of the laser light.

In the second aspect, at least one of the first irradiation step and the second irradiation step may include a preliminary irradiation step of irradiating the laser light having a relatively small intensity and a latter irradiation step of irradiating the laser light having a relatively large intensity, after the preliminary irradiation step.

In this case, it is possible to even more effectively prevent the melting, vaporization and extinction of the first wire and the second wire in the vicinity of the irradiation region of the laser light.

In the second aspect, at least one of the first irradiation step and the second irradiation step may irradiate the laser light intermittently a plurality of times.

In this case, it is possible to even more effectively prevent the melting, vaporization and extinction of the first wire and the second wire in the vicinity of the irradiation region of the laser light.

In the second aspect, of the plurality of intersection points, at a first intersection point where a first wire [n +1], which is adjacent to the first wire [n] to another side in the second direction, intersects the second wire [n], the first wire [n + 1] may be disposed to the one side in the third direction with respect to the second wire [n], and, of the plurality of intersection points, at a second intersection point where a second wire [n +1], which is adjacent to the second wire [n] to another side in the first direction, intersects the first wire [n], the second wire [n + 1] may be disposed to the one side in the third direction with respect to the first wire [n].

When the first wire [n] and the second wire [n] are bonded at the bonding intersection point [n], the catheter can suppress the first wire [n] from moving to the one side in the third direction, using the second wire [n + 1]. Further, when bonding the first wire [n] and the second wire [n] at the bonding intersection point [n], the catheter can suppress the second wire [n] from moving to the one side in the third direction, using the first wire [n + 1].

The second aspect may include:
a third irradiation step of, after the irradiation of the laser light by the second irradiation step, at a bonding intersection point [n + 1] where the first wire [n +1] and the second wire [n + 1] intersect each other, alloying and bonding the first wire [n + 1] and the second wire [n + 1] at the bonding intersection point [n + 1] and thermally cutting a section, of the second wire [n + 1], further to the one side in the second direction than the bonding intersection point [n + 1], by irradiating the laser light from the one side in the third direction onto a first irradiation region [n + 1] including at least a portion on the one side in the second direction of a bonding region [n + 1] overlapping the first wire [n + 1] and the second wire [n + 1] when the braided body is viewed from the one side in the third direction, and a portion of the second wire [n + 1], not overlapping the first wire [n + 1], and
a fourth irradiation step of, after the irradiation of the laser light by the third irradiation step, cutting the first wire [n + 1] at a position of a second irradiation region [n + 1], by irradiating the laser light from the one side in the third direction onto the second irradiation region [n + 1] separated to the one side in the first direction from the bonding intersection point [n + 1] of the first wire [n + 1].

In this case, the first wire [n + 1] and the second wire [n + 1] can be bonded and thermally cut.

In the second aspect, the bonding intersection point [n] and the bonding intersection point [n + 1] may be disposed to be aligned in the extension direction.

In this case, it is possible to cut the reinforcement member in a direction orthogonal to the extension direction.

In the second aspect, in the first irradiation step and the second irradiation step, the laser light may be irradiated in a state in which an inert gas in a laminar flow state is blown onto the reinforcement member.

In this case, it is thus possible to suppress the occurrence of burning by oil and oxidation of the first wire and the second wire resulting from the laser irradiation.

The second aspect may further include:
a preliminary step of disposing an inner layer tube having a circular cylindrical shape around a metal wire having a cylindrical pillar shape extending in the extension direction, and disposing the metal wire and the inner layer tube in a lumen of the reinforcement member, the laser light being irradiated, in the first irradiation step and the second irradiation step, onto the braided body of the reinforcement member prepared by the preliminary step, and
a latter step of disposing an outer layer tube having a circular cylindrical shape around the reinforcement member, after the irradiation of the laser light by the second irradiation step.

In this case, the catheter can be manufactured that is provided with the reinforcement member, the inner layer tube, and the outer layer tube. In particular, by using the metal wire, the catheter provided with the thin inner layer tube can be easily manufactured. Further, by using the metal wire, it is possible to suppress the laser light irradiated onto the reinforcement member from passing through the lumen and reaching the opposite side. As a result, it is possible to reduce a possibility of the reinforcement member on the opposite side being melted by the laser light.

In the second aspect, a light transmittance of the inner layer tube may be higher than a light transmittance of the outer layer tube.

In this case, it is possible to suppress the inner layer tube from generating heat due to absorbing the laser light. Thus, it is possible to reduce a possibility of the inner layer tube melting or being damaged due to heat generation as a result of the irradiation of the laser light, and a possibility of the first wire and the second wire melting and becoming bonded at a section other than the bonding portion.

In the second aspect, a thermal conductivity of the metal wire may be from 350 to 450 w/mK.

In other words, since thermal diffusion is promoted by using the material having the high thermal conductivity in the metal wire also, it is possible to further suppress a local temperature rise of the metal wire, by the thermal diffusion. As a result, it is possible to reduce a possibility of the inner layer tube melting due to the temperature rise in the metal wire.

### Brief description of the drawings

Fig. 1 is a view showing a catheter 1 and a connector 9.
Fig. 2 is a view showing the catheter 1.
Fig. 3 is a view showing a reinforcement member 2 in an unfolded state.
Fig. 4 is a perspective view of an enlarged portion of the reinforcement member 2 in the unfolded state.
Fig. 5 is a view showing a vicinity of a distal end portion of the reinforcement member 2 in the unfolded state.
Fig. 6 is a perspective view of an enlarged portion of Fig. 5.
Fig. 7 is a photo in which bonding portions 5 of the reinforcement member 2 are captured.
Fig. 8 is a photo in which a cross-section of a bonding portion 5[x] is captured.
Fig. 9 is a schematic view showing a cross section of a bonding portion bonded using a known method.
Fig. 10 is a flowchart showing a manufacturing method of the catheter 1.
Fig. 11 is a view showing a long wire material 10 prepared in a preliminary process.
Fig. 12 is a perspective view of an enlarged portion of the reinforcement member 2 in the unfolded state, in the course of manufacturing the catheter 1.
Fig. 13 is a view as seen from a first direction of the vicinity of a bonding intersection point Qc[n].
Fig. 14A is a view showing a process in which a bonding portion 5[n] is formed by irradiation of laser light.
Fig. 14B is a view showing the process in which the bonding portion 5[n] is formed by the irradiation of the laser light.
Fig. 15A is an explanatory view describing an output pattern of the laser light.
Fig. 15B is an explanatory view describing an output pattern of the laser light.
Fig. 16A is a view showing a process of thermally cutting a first wire 3[n] by the irradiation of the laser light.
Fig. 16B is a view showing the process of thermally cutting the first wire 3[n] by the irradiation of the laser light.
Fig. 17A is a view showing a state when thermally cutting the first wire 3[n].
Fig. 17B is a view showing a state when thermally cutting the first wire 3 [n].
Fig. 18 is a view showing a latter process of disposing an outer layer tube 7 is disposed.
Fig. 19A is an explanatory view describing an output pattern of the laser light in a modified example.
Fig. 19B is an explanatory view describing an output pattern of the laser light in a modified example.

### Description of Embodiments

### Overview of catheter 1

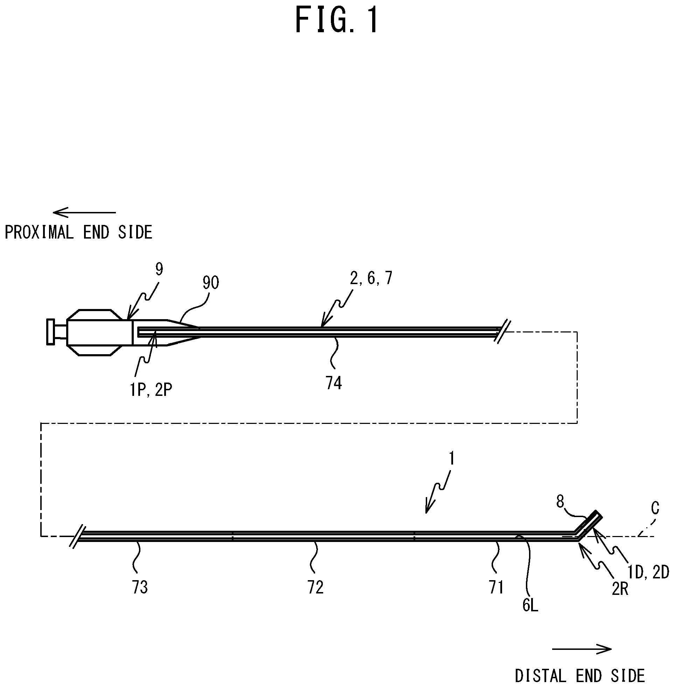

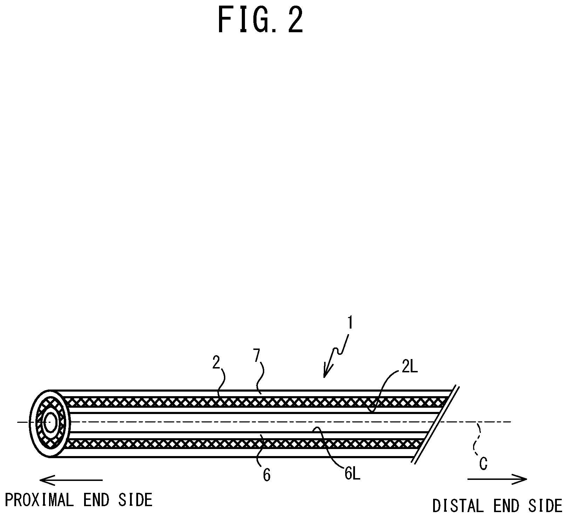

Hereinafter, an embodiment of the present disclosure will be described with reference to the drawings. As shown in Fig. 1 and Fig. 2, a catheter 1 includes a reinforcement member 2, an inner layer tube 6, and an outer layer tube 7. Hereinafter, a direction along the catheter 1 is referred to as an "extension direction." Of both end portions in the extension direction of the catheter 1, the end portion corresponding to one side is referred to as a "distal end portion 1D." Of both the end portions in the extension direction of the catheter 1, the end portion corresponding to the other side is referred to as a "proximal end portion 1P." The catheter 1 internally includes a lumen 6L for a wire or the like to be passed through. The lumen 6L extends in the extension direction between the distal end portion 1D and the proximal end portion 1P of the catheter 1. The connector 9 for passing the wire or the like through the catheter 1 is connected to the proximal end portion 1P of the catheter 1. A reinforcement tube 90 reinforces the vicinity of a connection portion between the proximal end portion 1P of the catheter 1 and the connector 9. In a plane orthogonal to the extension direction, a radial direction that uses a line passing through a cross-sectional center of the catheter 1 (referred to as a "center line C") as a reference, is referred to as a third direction. Of the third direction, a side in the vicinity of the center line C of the catheter 1 is referred to as an "inner side," and a side separated from the center line C of the catheter 1 is referred to as an "outer side."

### Reinforcement member 2

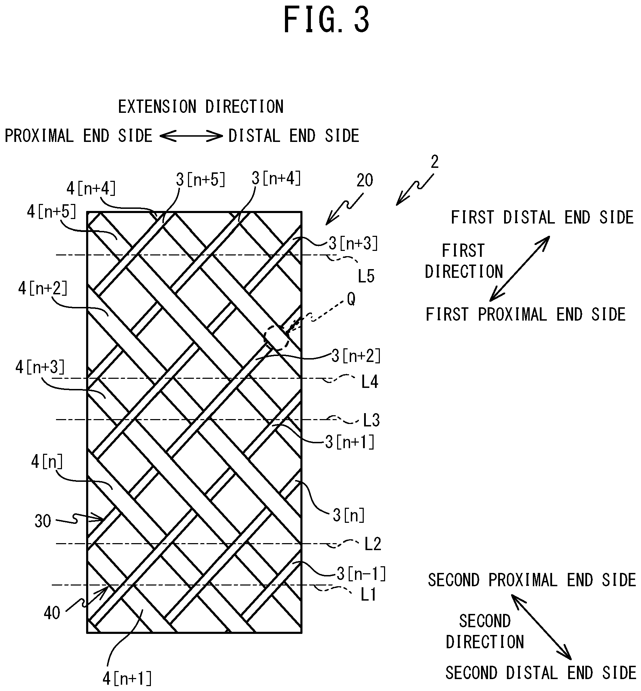

The reinforcement member 2 is a flexible tubular member. The reinforcement member 2 reinforces the strength in the extension direction of the catheter 1. The reinforcement member 2 extends from the distal end portion 1D to the proximal end portion 1P of the catheter 1 along the extension direction. The reinforcement member 2 is formed as a result of a braided body 20 (refer to Fig. 3), which is formed by a plurality of interwoven metal wires, being made into a circular cylindrical shape and both end portions thereof being cut. The center line C extends in the extension direction while passing through a center of the braided body 20.

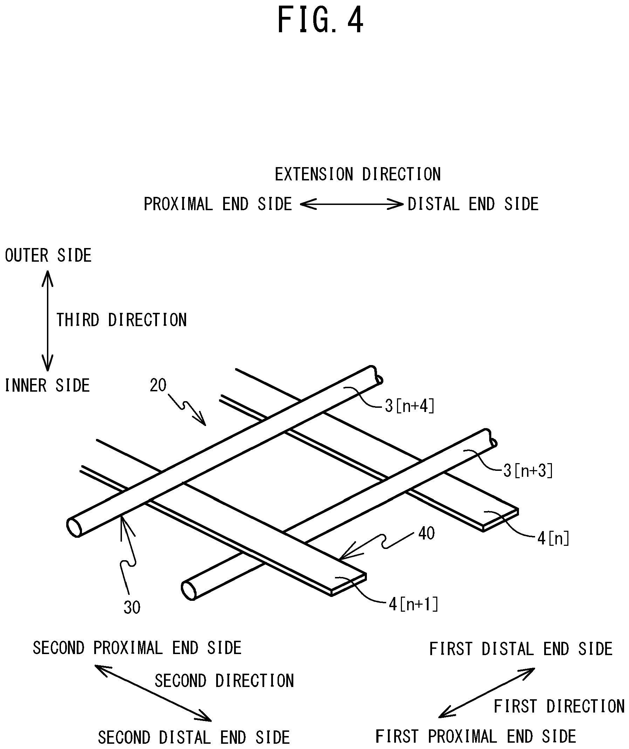

Fig. 3 and Fig. 4 show a portion of the braided body 20 in an unfolded state. The braided body 20 includes a plurality of first wires 30 and a plurality of second wires 40. The plurality of first wires 30 extend in a first direction that intersects the extension direction. The plurality of second wires 40 extend in a second direction that intersects the extension direction and the first direction. The braided body 20 is a so-called twill weave metal mesh that is formed of a wire net woven into a mesh form by the plurality of first wires 30 and the plurality of second wires 40 passing alternately over each other two wires at a time. As shown in Fig. 4, the direction orthogonal to the first direction and the second direction corresponds to the third direction. The third direction is orthogonal to the extension direction. A near side in Fig. 3 corresponds to the outer side in the third direction. A far side in Fig. 3 corresponds to the inner side in the third direction.

As shown in Fig. 3, the plurality of first wires 30 are respectively denoted as a first wire 3[n - 1], a first wire 3 [n], a first wire 3[n + 1], and so on, and these are generically referred to as first wires 3. The plurality of second wires 40 are respectively denoted as a second wire 4[n - 1], a second wire 4[n], a second wire 4[n + 1], and so on, and these are generically referred to as second wires 4. A plurality of positions at which the plurality of first wires 30 and the plurality of second wires 40 intersect are referred to as a plurality of intersection points Q. In Fig. 3, of the plurality of intersection points Q, a reference sign is assigned only to the intersection point Q at which the first wire 3[n + 2] and the second wire 4[n + 4] intersect each other, and the reference signs for the remaining plurality of intersection points Q are omitted.

Of the plurality of intersection points Q shown in Fig. 3, at the intersection points Q disposed at positions overlapping virtual lines L1, L2, L3, L4, and L5 extending in the extension direction, the first wire 3 is disposed to the outer side in the third direction (the near side in Fig. 3) with respect to the second wire 4.

The material of the first wire 3 is tungsten (W), and the melting point thereof is 3422°C. The cross-sectional shape of the first wire 3 is a perfect circle (refer to Fig. 4). The absorption coefficient of the first wire 3 is equal to or greater than 0.4. The material of the second wire 4 is stainless steel (SUS 304), and the melting point thereof is 1450°C. The cross-sectional shape of the second wire 4 is rectangular (refer to Fig. 4). The absorption coefficient of the second wire 4 is approximately 0.3. The difference between the melting points of the first wire 3 and the second wire 4 is approximately 2000°C. The melting point of the first wire 3 is at least 500°C higher than the melting point of the second wire 4. The absorption coefficient of the first wire 3 is greater than the absorption coefficient of the second wire 4. Note that, in order for the absorption coefficient of the first wire 3 to be greater than the absorption coefficient of the second wire 4, black wire is used for the first wire 3.

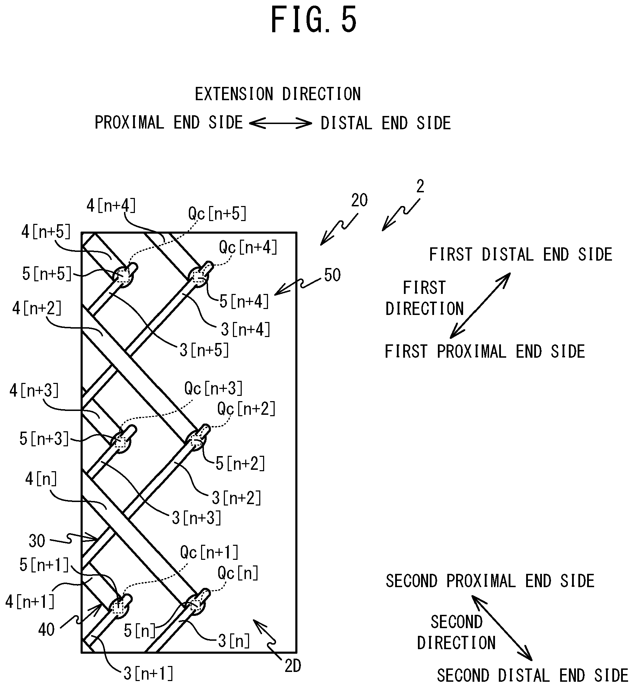

The end portion on the distal end side of the reinforcement member 2 shown in Fig. 1 (hereinafter referred to as a distal end portion 2D of the reinforcement member 2), and the end portion on the proximal end side (hereinafter referred to as a proximal end portion 2P of the reinforcement member 2 (refer to Fig. 1)) are respectively formed by cutting the braided body 20 using the irradiation of the laser light. Further, at the distal end portion 2D and the proximal end portion 2P, a plurality of bonding portions 50 (refer to Fig. 5) are formed on some of the plurality of intersection points Q between the plurality of first wires 30 and the plurality of second wires 40, so that the plurality of first wires 30 and the plurality of second wires 40 of the cut braided body 20 do not come apart.

As shown in Fig. 3 and Fig. 4, of both sides in the first direction, the side in the vicinity of the distal end portion 2D (refer to Fig. 1) is referred to as a "first distal end side," and the side in the vicinity of the proximal end portion 2P (refer to Fig. 1) is referred to as a "first proximal end." Of both sides in the second direction, the side in the vicinity of the distal end portion 2D (refer to Fig. 1) is referred to as a "second distal end side," and the side in the vicinity of the proximal end portion 2P (refer to Fig. 1) is referred to as a "second proximal end."

Fig. 5 is a view in which a portion of the distal end portion 2D of the reinforcement member 2 is enlarged. The plurality of bonding portions 50 are respectively denoted as a bonding portion 5[n], a bonding portion 5[n + 1], and so on, and these are generically referred to as a bonding portion 5. Of the plurality of intersection points Q, the intersection points at which the bonding portions 5 are formed are denoted as a bonding intersection point Qc[n], a bonding intersection point Qc[n + 1], and so on. A bonding portion 5[x] (x is one of n, n + 1, n + 2, n + 3, n + 4, or n + 5) is formed at a bonding intersection point Qc[x] between a first wire 3[x] and a second wire 4[x]. At the bonding intersection point Qc[x], the bonding portion 5[x] bonds the first wire 3[x] and the second wire 4[x], by alloying the first wire 3[x] and the second wire 4[x] at a contact portion therebetween.
More specifically, the alloy at the bonding portion 5[x] is a state that satisfies the following conditions.
(1) The first wire 3[x] and the second wire 4[x] are fused by the irradiation of the laser light.
(2) The components of each of the first wire 3[x] and the second wire 4[x] are mixed, and diffused, and after that, are caused to fuse and solidify.
(3) The whole of a boundary surface between the first wire 3[x] and the second wire 4[x] is not covered by an intermetallic compound layer.
(4) The first wire 3 [x] and the second wire 4[x] are not separated by a boundary created by the intermetallic compound layer of (3), a solid solution is also formed, and both the first wire 3[x] and the second wire 4[x] are favorably mixed together.
The bonding intersection point Qc[n] and the bonding intersection point Qc[n + 1], the bonding intersection point Qc[n + 2] and the bonding intersection point Qc[n + 3], and the bonding intersection point Qc[n + 4] and the bonding intersection point Qc[n + 5] are respectively disposed to be aligned in the extension direction.

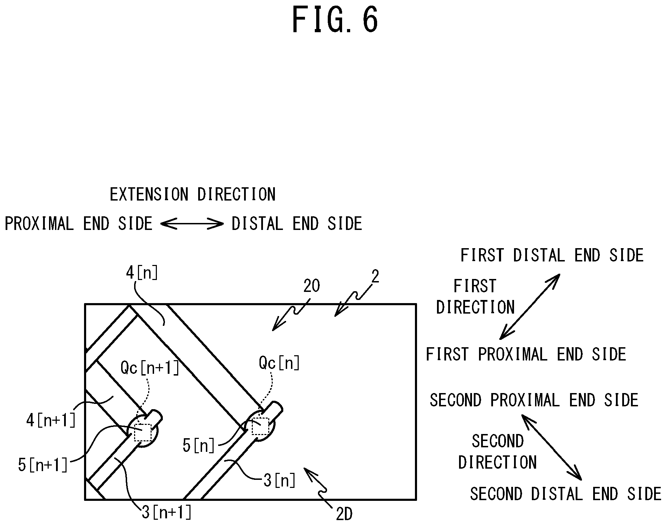

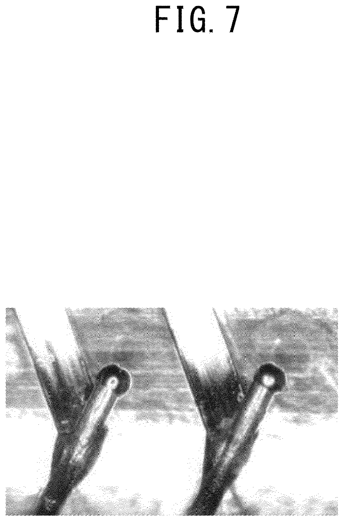

At the bonding intersection point Qc[x], the first wire 3[x] is disposed to the outer side (the near side in Fig. 5) with respect to the second wire [x]. As shown in Fig. 6 and Fig. 7, of the second wire 4[x], a section further to the second distal end side in the second direction than the bonding intersection point Qc[x] curves to the outer side in the third direction (the near side in Fig. 6 and Fig. 7), and is wound onto to the first wire 3[x]. The end portion of the first wire 3[x] to the first distal end side in the first direction is separated from the bonding intersection point Qc[x]. In other words, the first wire 3 [x] extends slightly from the bonding intersection point Qc[x] to the first distal end side in the first direction. A distance in the first direction between the end portion on the first distal end side of the first wire 3[x] and the bonding intersection point Qc[x] is a value in a range from 0.03 mm to 0.15 mm.

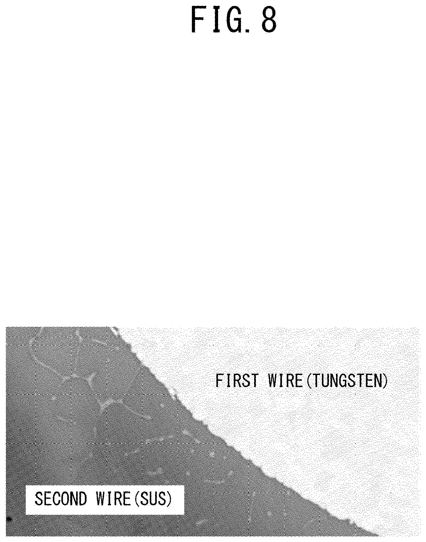

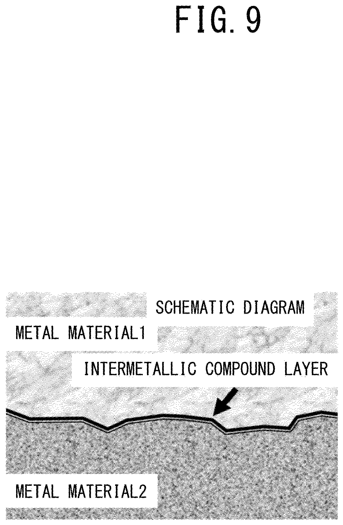

Fig. 8 is a cross-sectional view showing a state in which, at the bonding intersection point Qc[x], the first wire 3[x] and the second wire 4[x] are bonded by the bonding portion 5[x]. As shown in Fig. 8, at boundary surfaces of each of the first wire 3[x] and the second wire 4[x], a state was verified that the first wire 3[x] and the second wire 4[x] were mixed together by the formed solid solution and were recrystallized. Note that when a first wire (a metal material 1, such as steel or the like) and a second wire (a metal material 2, such as an aluminum alloy or the like) are bonded using a known general method, for example, the first wire and the second wire are separated by an intermetallic compound layer formed at each of boundary surfaces of the first wire and the second wire, as shown in Fig. 9. From this result, it was verified that the bonding portion 5[x] can more firmly bond the first wire 3[x] and the second wire 4[x] in comparison to known art.

### Inner layer tube 6, outer layer tube 7, soft tip 8

As shown in Fig. 2, the inner layer tube 6 has a circular cylindrical shape centered on the center line C, and is disposed in a lumen 2L of the reinforcement member 2. The material of the inner layer tube 6 is polytetrafluoroethylene (PTFE). The light transmittance of the inner layer tube 6 is equal to or greater than 90%. The lumen 6L of the inner layer tube 6 extends in the extension direction, and forms the lumen 6L of the catheter 1. The inner layer tube 6 is provided in order to separate the lumen 6L of the catheter 1 and the reinforcement member 2, and prevents a wire or the like that passes through the lumen 6L from becoming caught up on the reinforcement member 2.

The outer layer tube 7 has a circular cylindrical shape centered on the center line C, and covers the outer side of the reinforcement member 2. The material of the outer layer tube 7 is a colored resin material containing a pigment. The light transmittance of the outer layer tube 7 is less than 90%. The light transmittance of the outer layer tube 7 is more preferably from 10% to 20%. The light transmittance of the inner layer tube 6 is higher than the light transmittance of the outer layer tube 7.

As shown in Fig. 1, the outer layer tube 7 includes a first outer layer tube 71, a second outer layer tube 72, a third outer layer tube 73, and a fourth outer layer tube 74 each having a different rigidity. The first outer layer tube 71, the second outer layer tube 72, the third outer layer tube 73, and the fourth outer layer tube 74 are arrayed in the extension direction, and are aligned in that order from the distal end portion 1D to the proximal end portion 1P of the catheter 1. The rigidity of the outer layer tube 7 gradually increases in the order of the first outer layer tube 71, the second outer layer tube 72, the third outer layer tube 73, and the fourth outer layer tube 74.

A soft tip 8 is provided at the distal end portion 1D of the catheter 1. The soft tip 8 comes into contact with each of the vicinity of the end portion on the distal end side of the side surface of the inner layer tube 6, and the vicinity of the distal end portion 2D of the reinforcement member 2. The soft tip 8 is fixed to the inner layer tube 6 and the reinforcement member 2 by welding, and terminates the distal end portion 1D of the catheter 1.

A curved portion 2R is provided in the vicinity of the distal end portion 1D of the catheter 1. The curved portion 2R is formed as a result of each of the reinforcement member 2, the inner layer tube 6, and the outer layer tube 7 being curved with respect to the extension direction.

### Manufacturing method of catheter 1

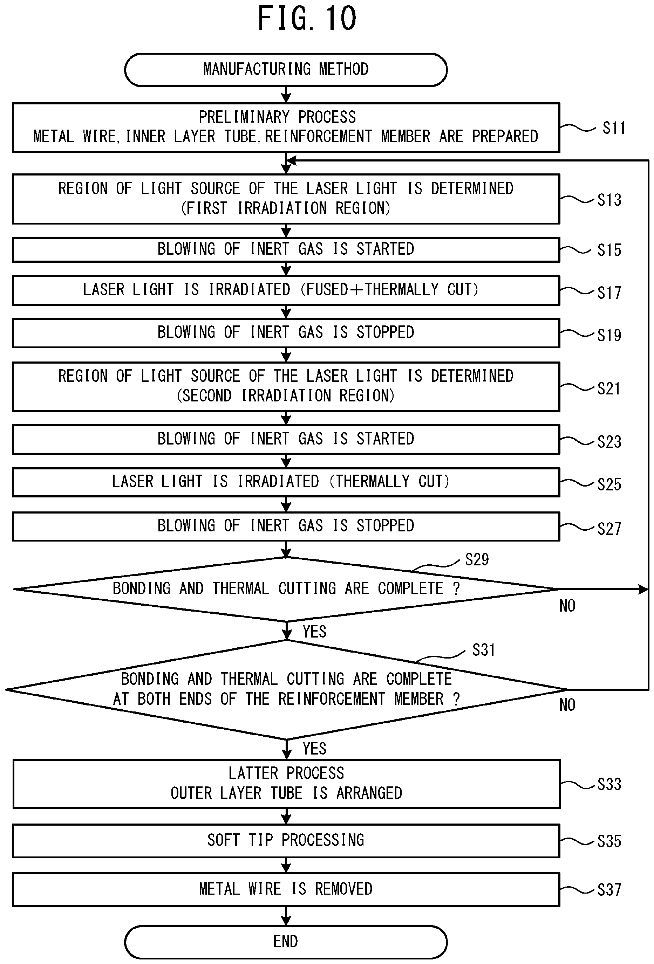

A manufacturing method of the catheter 1 will be described with reference to Fig. 10. In the present embodiment, the catheter 1 is formed as a result of both ends in the extension direction of the reinforcement member 2 being bonded and thermally cut by the irradiation of laser light, and the distal end portion 2D and the proximal end portion 2P being formed. It is assumed that first, the distal end of the reinforcement member 2 is cut by the irradiation of the laser light and the distal end portion 2D is formed, and next, the proximal end of the reinforcement member 2 is cut by the irradiation of the laser light and the proximal end portion 2P is formed.

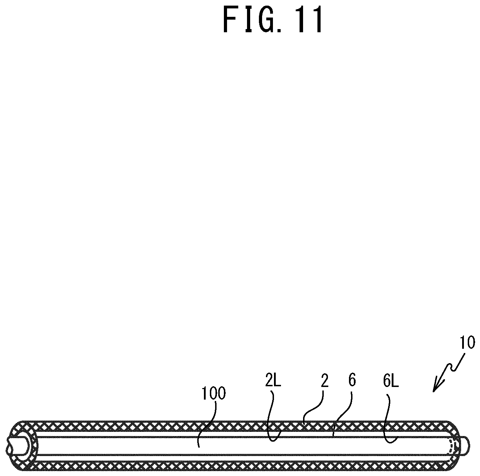

First, a preliminary process is performed (step S11). In the preliminary process, a metal wire 100, the inner layer tube 6, and the reinforcement member 2 (refer to Fig. 11) are prepared. This is described in more detail as follows.

A long wire material 10 wound onto a bobbin is pulled out. As shown in Fig. 11, a necessary length of the pulled out long wire material 10 is roughly cut, using scissors or the like. The long wire material 10 has a configuration in which the inner layer tube 6 disposed around the cylindrically shaped metal wire 100 is disposed in the lumen 2L of the reinforcement member 2. In Fig. 11, in order to simplify understanding, the inner layer tube 6 and the metal wire 100 disposed in the lumen 2L of the reinforcement member 2 are shown to be visible. The metal wire 100 is made of flexible copper. The thermal conductivity of the metal wire 100 is from 350 to 450 w/mK. Through thermal diffusion, the metal wire 100 can suppress a local temperature increase when the laser light is irradiated in the manufacturing process. The surface of the metal wire 100 is plated with a silver color, and reflects the laser light irradiated in the manufacturing process. The inner layer tube 6 is formed of resin, as a coating for the surface of the metal wire 100. The light transmittance of the inner layer tube 6 is equal to or greater than 90%, and transmits the laser light irradiated in the manufacturing process. The reinforcement member 2 is disposed on the outer side of the inner layer tube 6, and is exposed. Further, in the preliminary process, in order to remove machine oil and the like attached to the plurality of first wires 30 and the plurality of second wires 40 (refer to Fig. 3) when manufacturing the reinforcement member 2, degreasing is performed with respect to the long wire material 10, using an organic solvent.

As shown in Fig. 10, after the preliminary process at step S11, a laser light irradiation region is determined, in order to bond the first wire 3 and the second wire 4 using the bonding portion 5 and to cut the second wire 4 in the vicinity of the bonding portion 5 at the distal end of the long wire material 10. Next, a position of a light source of the laser light is determined such that the laser light is irradiated onto the determined irradiation region (step S13). A case is assumed in which, at the distal end of the reinforcement member 2, the bonding portions 5[x] are sequentially formed in correspondence to each time x changes in order of n, n + 1, n + 2, n + 3, and so on, and the distal end portion 2D is formed.

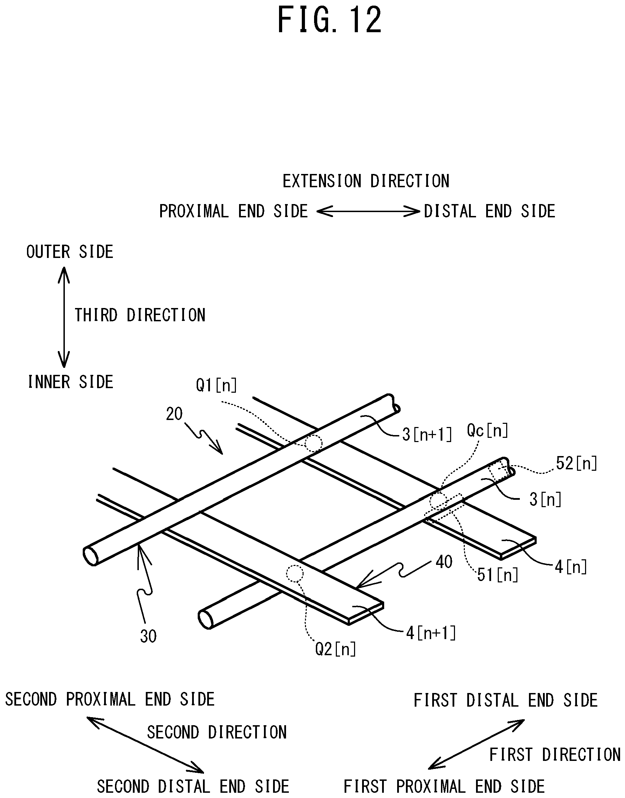

A case will be described when x = n, that is, when the first wire 3[n] and the second wire [n] are bonded by the bonding portion 5[n] at the bonding intersection point Qc[n] shown in Fig. 5, and the second wire 4[n] is cut. Note that, as shown in Fig. 12, at the bonding intersection point Qc[n], the first wire 3[n] is disposed to the outer side in the third direction with respect to the second wire 4[n]. At a bonding intersection point Q1 [n] at which the first wire 3 [n + 1], which is adjacent to the first wire 3 [n] on the second proximal end side in the second direction, intersects the second wire 4[n], the first wire 3[n + 1] is disposed to the outer side in the third direction with respect to the second wire 4[n]. At a bonding intersection point Q2[n] at which the second wire 4[n + 1], which is adjacent to the second wire 4[n] on the first proximal end side in the first direction, intersects the first wire 3[n], the second wire 4[n + 1] is disposed to the outer side in the third direction with respect to the first wire 3 [n].

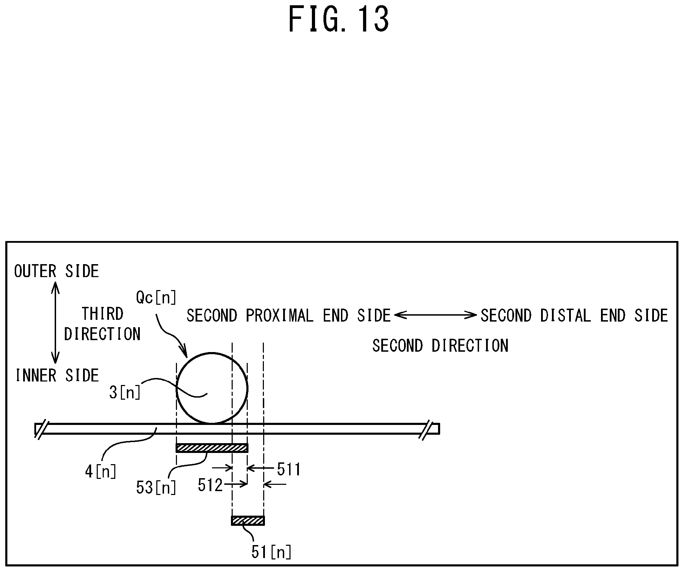

The irradiation region of the laser light whose position is determined at step S13 is referred to as a first irradiation region 51[n]. Specifically, the first irradiation region 51[n] is prescribed as a region described below. As shown in Fig. 13, at the bonding intersection point Qc[n], a bonding region 53 [n] is defined in which the first wire 3[n] overlaps the second wire 4[n] when seen from the outer side of the braided body 20 in the third direction. In this case, the first irradiation region 51[n] includes at least a partial section 511 of the second distal end side in the second direction of the bonding region 53[n], and a section 512 that is a section, of the second wire 4[n] further to the second distal end side than the bonding region 53[n] and is a section that does not overlap with the first wire 3 [n].

Note that the determining of the position of the light source of the laser light is performed using an image system. The image system can display a center and peripheral edges of an optical path of the laser light determined in accordance with the position of the light source. A diameter of a circular shape drawn by the peripheral edges of the optical path of the laser light (a spot diameter of the laser light) is set to be equal to or less than a length of the shorter of the diameter of the first wire 3 and the width of the second wire 4.

As shown in Fig. 10, after determining the position of the light source of the laser light at step S13, blowing of an inert gas in a laminar flow state is started toward the vicinity of the first irradiation region 51[n] (refer to Fig. 13) of the braided body 20 (step S15). The inert gas is, more specifically, argon gas or helium gas. Next, the laser light is output from the light source, in the state in which the inert gas is being blown. In this way, the laser light is irradiated onto the first irradiation region 51[n] from the outer side in the third direction (step S 17).

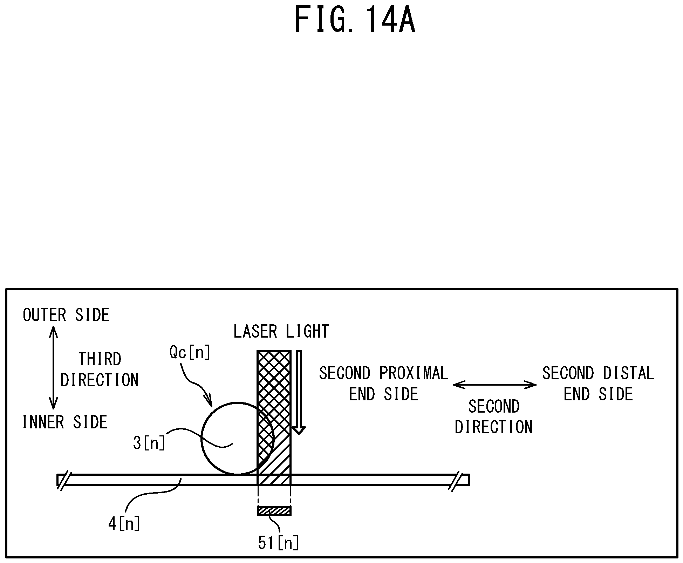

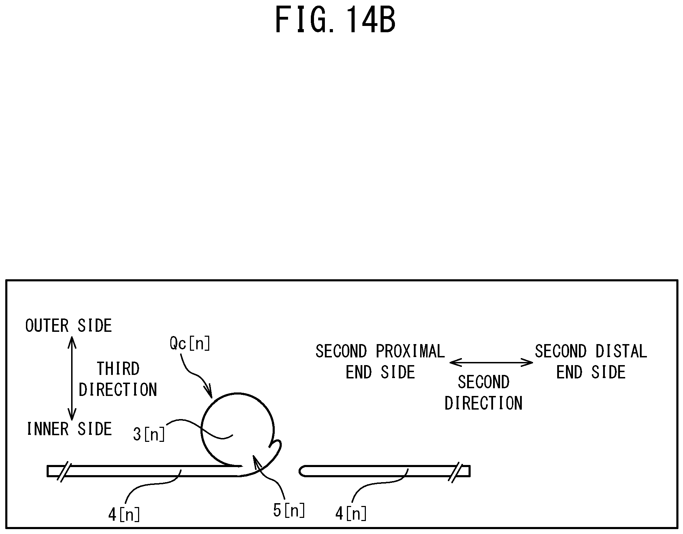

When the laser light is irradiated onto the first irradiation region 51[n] at step S 17, as shown in Fig. 14A, the laser light is first irradiated onto the first wire 3[n]. In this way, the energy of the laser light is imparted to the first wire 3[n], and the first wire 3[n] is heated and fused. Further, the energy of the laser light decreases as a result of being imparted to the first wire 3 [n]. The laser light is next irradiated onto the second wire 4[n]. In this way, the energy of the laser light is imparted to the second wire 4[n], and the second wire 4[n] is heated and fused, and is thermally cut. As a result of this, as shown in Fig. 14B, the first wire 3[n] and the second wire 4[n] come into contact at a mutual fusion section, and become alloyed, and are thus bonded. Further, the second wire 4[n] is thermally cut at a section further to the second distal end side in the second direction than the bonding portion 5[n], curves to the outer side in the third direction and winds around the first wire 3[n] (refer to Fig. 6 and Fig. 7). After the first wire 3[n] and second wire 4[n] are bonded by the bonding intersection point Qc[n], the irradiation of the laser light from the light source is stopped.

Note that, at step S17, the bonding of the first wire 3[n] and the second wire 4[n] and the thermal cutting of the second wire 4[n] are realized by one pulse of the irradiation of laser light. Thus, compared to when each of the processes are performed separately, displacement is unlikely to occur between the position of the bonding intersection point Qc[n] and the thermal cutting position of the second wire 4[n].

Further, in the course of bonding the first wire 3[n] and the second wire 4[n] by the irradiation of laser light, and thermally cutting the second wire 4[n], there is a possibility that the first wire 3 and the second wire 4 may generate heat, due to the irradiation of the laser light, at a position different from the bonding intersection point Qc[n]. In this case, there is a possibility that the physical properties of the first wire 3 and the second wire 4 may change due to heat, or thermal cutting may occur in a region different from the bonding intersection point Qc[n], and this is not preferable. In contrast to this, in the present embodiment, irradiation is performed while changing the energy imparted to the braided body 20 by the irradiation of the laser light over time. In this way, the possibility of the first wire 3 and the second wire 4 generating heat due to the irradiation of the laser light at a position different from the bonding intersection point Qc[n] is reduced. This is described in more detail as follows.

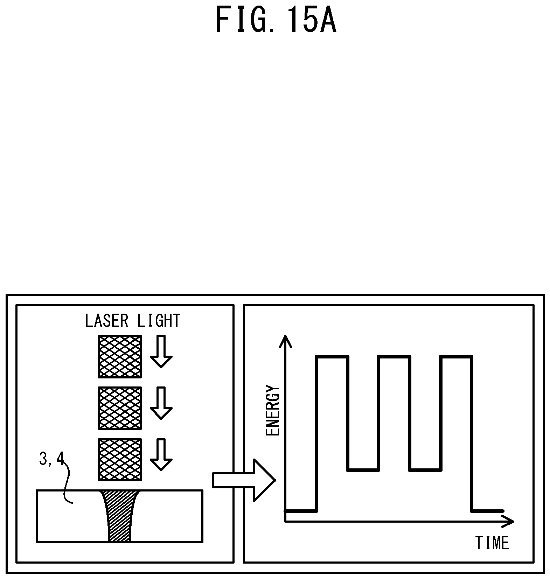

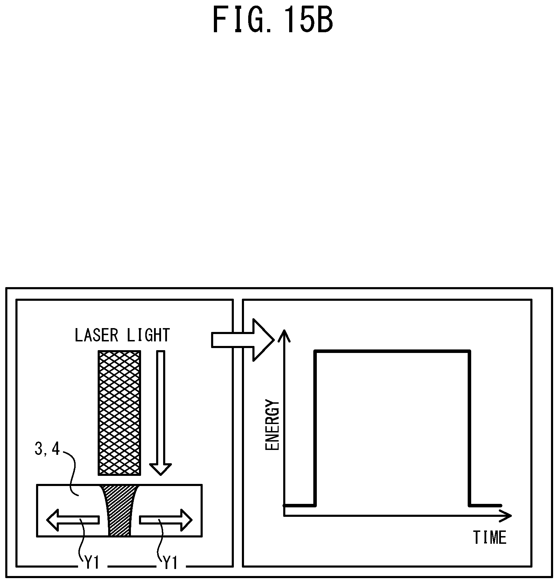

As shown in Fig. 15A, at step S17, the one pulse of laser light is divided up and intermittently irradiated a plurality of times. More specifically, the laser light is irradiated while being alternately and repeatedly switched between a period in which the intensity of the laser light is relatively large (referred to as a "high level period") and a period in which the intensity of the laser light is relatively small (hereinafter referred to as a "low level period"). In this case, the heat generated by the energy of the laser light in the first wire 3 and the second wire 4 of the braided body 20 is not easily diffused to the surroundings. The reason for this is that the heat cools during the low level period in which the intensity of the irradiated laser light is relatively small, and the diffusion of the heat to the surroundings is suppressed. This effect is generally called a divided-pulse effect (cool effect). In this case, as shown in Fig. 15B, it is possible to suppress the diffusion of the heat to the surroundings (arrows Y1) resulting from the one pulse of laser light being continuously irradiated at a constant intensity.

Note that the intensity and an irradiation period of time of the laser light are adjusted such that it is possible to impart the energy necessary to thermally cut the first wire 3 using the energy of one pulse. Note also that it is sufficient that the intensity of the laser light in the low level period be equal to or lower than half the intensity of the laser light in the high level period. In other words, it is sufficient that the intensity of the laser light in the low level period be any value from 0% to 50% the intensity of the laser light in the high level period. Further, when the intensity of the laser light in the low level period is zero, this means that the irradiation of the laser light in the low level period is stopped. Further, the period of time of the low level period is one of a value from 10% to 100% of the time period of the high level period.

As shown in Fig. 10, after the irradiation of the laser light onto the first irradiation region 51[n] at step S17 has ended, the blowing of the inert gas started at step S15 is stopped (step S 19). Next, an irradiation region of the laser light irradiated in order to cut the first wire 3[n] in the vicinity of the bonding portion 5[n] is determined. Note that, of the first wire 3[n] shown in Fig. 5, the first wire 3[n] is thermally cut at a position separated to the first distal end side in the first direction with respect to the bonding intersection point Qc[n].

Next, the position of the light source of the laser light is determined such that the laser light is irradiated onto the determined irradiation region (step S21). As shown in Fig. 12, the irradiation region of the laser light for which the position is determined at step S21 is referred to as a second irradiation region 52[n]. As shown in Fig. 10, after determining the position of the light source of the laser light at step S21, the blowing of the inert gas in a laminar flow state is started toward the vicinity of the second irradiation region 52[n] of the braided body 20 (step S23). Next, the laser light is output from the light source, in the state in which the inert gas is being blown. In this way, the laser light is irradiated onto the second irradiation region 52[n] from the outer side in the third direction (step S25).

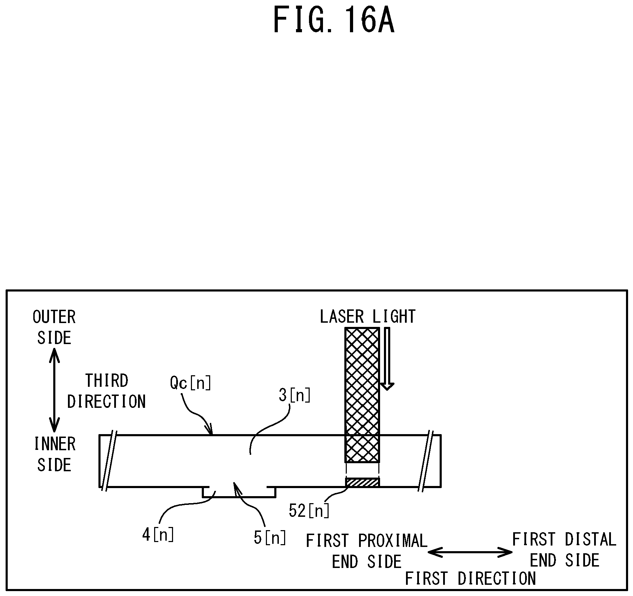

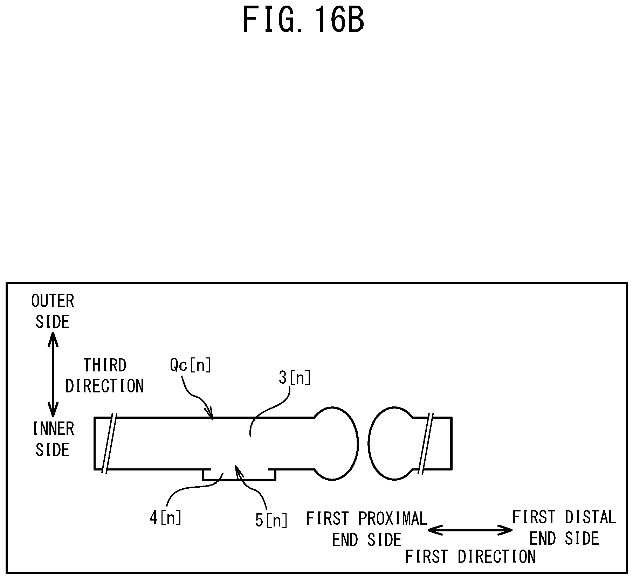

As shown in Fig. 16A, when the laser light is irradiated at step S25, the laser light imparts energy to the second irradiation region 52[n] of the first wire 3 [n]. As a result of this, as shown in Fig. 16B, the first wire 3[n] is thermally cut further to the first distal end side in the first direction than the bonding portion 5[n]. After the first wire [n] is thermally cut, the irradiation of the laser light from the light source is stopped.

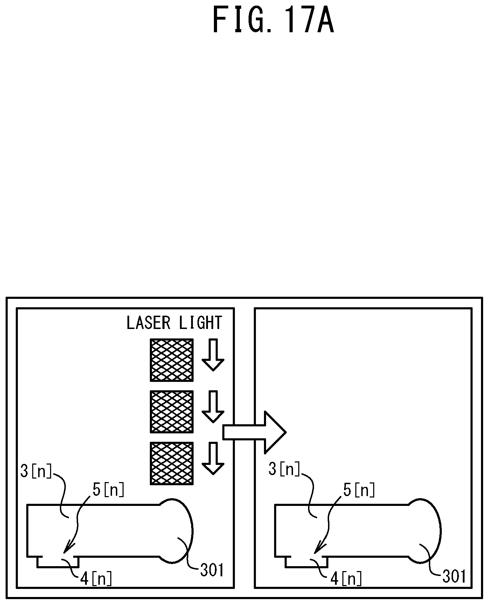

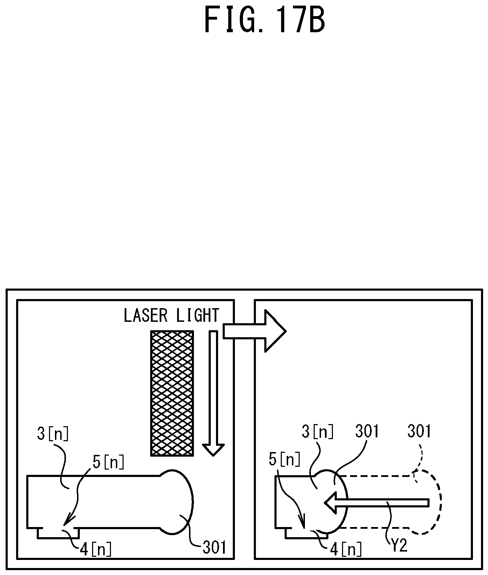

Note that irradiation conditions of the laser light irradiated onto the second irradiation region 52[n] at step S23 are the same as the irradiation conditions of the laser light irradiated onto the first irradiation region 51[n] at step S17. In other words, the one pulse of laser light is divided up and intermittently irradiated the plurality of times. Thus, the heat generated by the energy of the laser light in the first wire 3[n] is not easily diffused to the surroundings. As a result, as shown in Fig. 17A, a thermally cut portion 301, of the first wire 3[n], that has been thermally cut by the irradiation of the laser light does not move after the irradiation of the laser light is stopped, and the position immediately after the irradiation of the laser light is maintained. Therefore, as shown in Fig. 17B, for example, it is possible to suppress the diffusion of the heat to the surroundings resulting from the one pulse of laser light being continuously irradiated at a constant intensity, and thus suppress the thermally cut portion 301 of the first wire 3[n] from moving toward the bonding portion 5[n] (an arrow Y2).

As described above, the state of separation of the thermally cut portion 301 of the first wire 3[n] and the second wire 4[n] is maintained, and thus, the heat of the thermally cut portion 301 of the first wire 3[n] is not transferred to the second wire 4[n]. Thus, even when the laser light is irradiated onto the second irradiation region 52[n], the composition of the second wire 4[n] does not change, apart from a portion at the bonding portion 5[n]. In other words, apart from at the bonding portion 5[n], the composition of the second wire 4[n] is the same, regardless of a distance from the bonding portion 5[n].

As shown in Fig. 10, after the irradiation of the laser light onto the second irradiation region 52[n] at step S25 has ended, the blowing of the inert gas started at step S23 is stopped (step S27).

Next, it is judged whether the bonding and the thermal cutting are complete for all the first wires 3 and the second wires 4 at the distal end of the reinforcement member 2 (step S29). When the bonding and the thermal cutting are not all complete (no at step S29), the processing returns to step S13. x is updated from n to n + 1, and step S13 to step S27 are repeated. In this way, as shown in Fig. 5, at the bonding intersection point Qc[n + 1], the first wire 3[n + 1] and the second wire 4[n + 1] are bonded by the bonding portion 5[n + 1], and the second wire 4[n + 1] is cut (step S13 to step S19). Further, the first wire 3[n + 1] is thermally cut at a position, of the first wire 3[n + 1] separated to the first distal end side in the first direction with respect to the bonding intersection point Qc[n + 1] (step S21 to step S27). At this time, the bonding portion 5[n] and the bonding portion 5[n + 1] are aligned in the extension direction.

The above-described processing is repeatedly performed while updating x in the order of n + 2 and n + 3. In this way, as shown in Fig. 5, at the bonding intersection point Qc[n + 2], the first wire 3[n + 2] and the second wire 4[n + 2] are bonded by the bonding portion 5[n + 1], and the second wire 4[n + 2] is cut (step S13 to step S19). Further, the first wire 3[n + 2] is thermally cut at a position, of the first wire 3[n + 2] separated to the first distal end side in the first direction with respect to the bonding intersection point Qc[n + 2] (step S21 to step S27). Further, at the bonding intersection point Qc[n + 3], the first wire 3[n + 3] and the second wire 4[n + 3] are bonded by the bonding portion 5[n + 3], and the second wire 4[n + 3] is cut (step S13 to step S19). Further, the first wire 3[n + 3] is thermally cut at a position, of the first wire 3[n + 3] separated to the first distal end side in the first direction with respect to the bonding intersection point Qc[n + 3] (step S21 to step S27). At this time, the bonding portion 5[n + 2] and the bonding portion 5[n + 3] are aligned in the extension direction.

The same processing as that described above is repeatedly performed while updating x in the order n + 4 and n + 5. In this way, the bonding portion 5[n +4] and the bonding portion 5[n + 5] shown in Fig. 5 are formed. Note that the bonding portions 5[n +4] and 5[n + 5] are aligned in the extension direction. Further, the bonding portions 5[n], 5[n + 2], and 5[n + 4], and the bonding portions 5[n + 1], 5[n + 3], and 5[n + 5] are respectively aligned with each other in the direction orthogonal to the extension direction. In this way, at the distal end of the reinforcement member 2, the reinforcement member 2 is cut in the direction orthogonal to the extension direction, and the distal end portion 2D is formed.

As shown in Fig. 10, when it is judged that the bonding and thermal cutting of all the first wires 3 and the second wires 4 at the distal end of the reinforcement member 2 is complete (yes at step S29), the processing advances to step S31. It is judged whether the bonding and the thermal cutting are complete for all the first wires 3 and the second wires 4 at both the distal end and the proximal end of the reinforcement member 2 (step S31). When the bonding and the thermal cutting are complete only at the distal end of the reinforcement member 2 and the bonding and the thermal cutting are not complete at the proximal end (no at step S31), the processing returns to step S13. Next, the same process as that for the distal end portion is performed with respect to the first wires 3 and the second wires 4 at the proximal end of the reinforcement member 2 (step S13 to step S29). In this way, the proximal end of the reinforcement member 2 is cut, and the proximal end portion 2P (refer to Fig. 1) is formed. Since both the ends of the reinforcement member 2 are cut and the distal end portion 2D and the proximal end portion 2P are formed (yes at step S31), the processing advances to step S33.

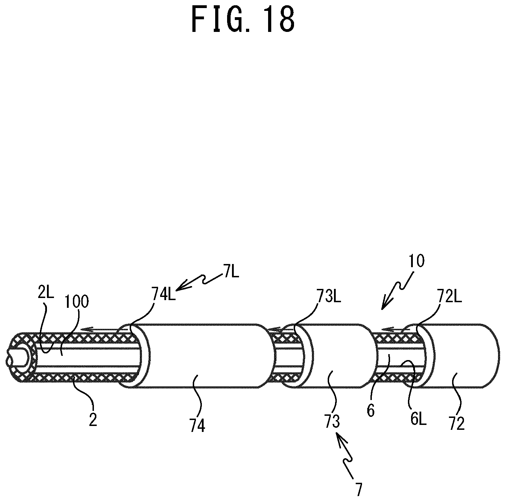

Next, a latter process is performed to dispose the outer layer tube 7 around the reinforcement member 2 (step S33). As shown in Fig. 18, the metal wire 100, the inner layer tube 6, and the reinforcement member 2 are passed in order through a lumen 74L of the fourth outer layer tube 74, a lumen 73L of the third outer layer tube 73, and a lumen 72L of the second outer layer tube 72. Note that, although not shown in Fig. 18, the metal wire 100, the inner layer tube 6, and the reinforcement member 2 are also passed through a lumen of the first outer layer tube 71. In the outer layer tube 7, the first outer layer tube 71 (refer to Fig. 1), the second outer layer tube 72, the third outer layer tube 73, and the fourth outer layer tube 74 are arranged in order from the distal end portion 2D toward the proximal end portion 2P (refer to Fig.1) of the reinforcement member 2.

As shown in Fig. 10, after the latter process (step S33) has ended, the distal end portion 2D of the reinforcement member 2 is subject to termination processing, using the soft tip 8 (step S35). Finally, the metal wire 100 is removed from the lumen 6L of the inner layer tube 6 (step S37). The manufacturing processing of the catheter 1 is completed in the manner described above.

### Method of using catheter 1

An example of a method of using the catheter 1 will be described. First, a user performs shaping of the distal end of the catheter 1, as necessary. The user is a doctor or the like. Next, the lumen 6L of the catheter 1 is passed around a guide wire inserted in advance into the blood vessel. The user applies a force to the proximal end side of the catheter 1 and pushes the catheter 1 bit by bit into the blood vessel from the distal end side. Note that the user rotates the catheter 1 as necessary and causes the distal end of the catheter 1 to be oriented in a desired direction. In this way, the user causes the distal end of the catheter 1 to reach a target section inside the blood vessel. After that, the user pulls out the guide wire from the catheter 1. In this state, as necessary, the user injects a contrast medium from the connector 9, inserts an embolus material, or the like.

### Actions and effects of present embodiment

In the catheter 1, the first wires 3[x] and the second wires 4[x] are alloyed at each of the contact portions therebetween, and the bonding portions 5[x] are formed as a result of bonding the first wires 3[x] and the second wires 4[x]. Thus, when the braided body 20 formed from the plurality of first wires 30 and the plurality of second wires 40 having the melting points that differ by 500°C or more is used as the reinforcement member 2, the catheter 1 can make it difficult for the end portions in the extension direction of the reinforcement member 2 to come apart.

The reinforcement member 2 is curved at the curved portion 2R. Even when a force acts on the reinforcement member 2 due to the curving of the curved portion 2R, the bonding portions 5[x] of the reinforcement member 2 can maintain the state in which the first wires 3[x] and the second wires 4[x] are bonded to each other.

Of the second wire 4[x], a section further to the second distal end side than the bonding intersection point Qc[x] curves to the outer side in the third direction, and is wound onto the first wire 3[x] (refer to Fig. 6 and Fig. 7). In this case, the catheter 1 can even more firmly bond the first wire [x] and the second wire [x].

The catheter 1 is provided with the inner layer tube 6. The inner layer tube 6 can suppress the wire or the like from becoming caught up on the reinforcement member 2, when the wire or the like is passed through the lumen 2L of the reinforcement member 2. Further, the catheter 1 is provided with the outer layer tube 7 that covers the reinforcement member 2 from the outer side. The outer layer tube 7 can suppress the reinforcement member 2 from being exposed. Thus, the catheter 1 can reduce a possibility of the reinforcement member 2 becoming caught up on the blood vessel when passing through the inside of the blood vessel.

The light transmittance of the inner layer tube 6 is higher than the light transmittance of the outer layer tube 7. In this case, when bonding the first wire 3[x] and the second wire 4[x] using the irradiation of the laser light, the inner layer tube 6 can suppress the generation of heat due to absorption of the laser light. Thus, the catheter 1 can reduce a possibility of the inner layer tube 6 generating heat at the time of the irradiation of the laser light, and thus suppress a possibility of the plurality of first wires 30 and the plurality of second wires 40 from being subject to the heat at portions other than the bonding portions 5[x] and deforming. Further, as a result of using polytetrafluoroethylene as the material of the inner layer tube, the inner layer tube 6 having the high light transmittance can be easily realized.

The first wire 3[x] is disposed on the outer side in the third direction with respect to the second wire 4[x]. In this case, in the manufacturing process of the catheter 1, the laser light from the light source disposed to the outer side in the third direction is appropriately irradiated onto the first wire 3[x], and the first wire 3[x] and the second wire 4[x] can be appropriately bonded. Further, at the time of the irradiation of the laser light, a larger amount of energy of the laser light is imparted to the first wire 3[x] having the higher melting point, and the first wire 3[x] can be fused. Further, the imparting of the energy of the laser light to the second wire 4[x] having the lower melting point can be suppressed and the second wire 4[x] can be fused, and thermally cut.

The cross-sectional shape of the first wire 3 is a perfect circle, and the cross-sectional shape of the second wire 4 is rectangular. In this case, before the first wires 3[x] and the second wires 4[x] are bonded, a gap can be formed between each of them. Thus, the first wires 3[x] and the second wires 4[x] can easily be caused to fuse and come into contact at the portions at which the respective gaps are formed and the first wires 3[x] and the second wires 4[x] can be easily alloyed. As a result, the first wires 3[x] and the second wires 4[x] can be alloyed at the respective contact portions, and can easily cause the bonding portions 5[x] to be formed.

The absorption coefficient of the plurality of first wires 30 is greater than that of the plurality of second wires 40. In this case, the first wire 3[x] having the relatively high melting point and the second wire 4[x] having the relatively low melting point are fused and alloyed at the same time, and also, the process to thermally cut the second wire [x] can be realized by the one-time irradiation of the laser light.

Apart from the bonding portion 5[x], the composition of the second wire 4[x] does not change even when irradiating the laser light for thermally cutting the first wire [x]. As a result, the composition of sections of the second wire 4[x] apart from the bonding portion 5[x] is the same, regardless of the distance from the bonding portion 5[x]. Thus, since the composition of the second wire 4 can be maintained to be stable, the catheter 1 can maintain the strength of the braided body 20.

The material of the plurality of first wires 30 is tungsten and the material of the plurality of second wires 40 is stainless steel (SUS). In this case, the catheter 1 can realize a favorable pushing in performance as a result of the high degree of hardness of the tungsten. Further, since the tungsten has superior shielding properties for radiation, verifying the position of the catheter 1 inside the body can be easily performed using radiation exposure.

When the long wire material 10 is cut at a desired section for manufacturing the catheter 1, there is a possibility that end portions of the first wire 3 and the second wire 4 may come apart due to elasticity. Further, when tungsten is used as the first wire 3, since the degree of hardness is high, although the pushing in performance of the catheter 1 is improved, the first wire 3 cannot easily be cut using a normal blade.

In contrast to this, in the manufacturing method of the catheter 1 shown in Fig. 10, the irradiation of the laser light onto the first irradiation region 51[x] at step S13 first imparts energy to a part of the first wire 3[x] and fuses the first wire 3[x]. At the same time, the laser light imparts energy to the second wire [x], fuses the second wire [x], thermally cuts the second wire [x], and also alloys and bonds the melted first wire 3[x] and the second wire 4[x]. The laser light irradiated onto the second irradiation region 52[x] at step S25 imparts energy to the first wire 3[x] and thermally cuts the first wire 3[x]. The above-described processes are repeated until both the ends of the reinforcement member 2 are cut and the distal end portion 2D and the proximal end portion 2P are formed. In this way, the distal end portion 2D and the proximal end portion 2P of the reinforcement member 2 do not easily come apart, due to the bonding portions 5[x].

When the bonding of the first wire 3[x] and the second wire 4[x], and the cutting of the second wire 4[x] are realized using separate irradiation of laser light, depending on the displacement of the position of the second wire 4[x] due to the irradiation of the first laser light, the position of a thermal cut portion of the second wire 4[n] is displaced from a target position. In this case, a positional displacement between the bonding portion 5[n] and the thermal cut portion of the second wire 4[n] is possible. In contrast to this, in the present embodiment, the bonding due to the alloying between the first wire 3[x] and the second wire 4[x], and the thermal cutting of the second wire 4[x] are performed at the same time by the one-time irradiation of the laser light at step S13. As a result, the possibility of the positional displacement between the bonding portion 5[x] and the thermal cut position of the second wire 4[x] can be reduced, and thus, accuracy of the positional relationship between the bonding portion 5[x] and the thermal cut position of the second wire 4[x] can be favorably maintained.

The energy of the laser light irradiated at step S13 is imparted first to the first wire 3[x], and the remaining energy is imparted to the second wire 4[x]. Thus, even when the melting point of the first wire 3 is 500°C or more than the melting point of the second wire 4, the bonding of the first wire 3[x] and the second wire 4[x], and the thermal cutting of the second wire 4[x] can be efficiently performed by the one-time irradiation of the laser light.

For example, when the bonding of the first wire 3[x] and the second wire 4[x] is performed after the thermal cutting of the first wire 3[x], there is a possibility that the first wire 3[x] may move at the time of the thermal cutting, and the positional relationship between the first wire 3[x] and the second wire [x] may be displaced. In this case, there is a possibility that the bonding between the first wire 3 [x] and the second wire 4[x] cannot be accurately performed. In contrast to this, in the present embodiment, the thermal cutting of the first wire 3[x] is performed after the bonding between the first wire 3[x] and the second wire 4[x]. In this case, the position of the first wire 3[x] with respect to the second wire 4[x] is unlikely to become displaced, due to the fact that the first wire 3[x] and the second wire 4[x] are bonding. Thus, the bonding between the first wire 3[x] and the second wire 4[x] and the cutting of the first wire 3 [x] can be accurately performed.

In the present embodiment, the energy imparted to the braided body 20 by the irradiation of the laser light at step S13 and step S25 changes over time. More specifically, at the time of the irradiation of the laser light at step S13 and step S25, the laser light is intermittently irradiated the plurality of times. In this case, it is possible to suppress the heat from being transmitted from the irradiation region of the laser light in the first wire 3 and the second wire to the surroundings. In this case, it is possible to prevent the occurrence of melting, vaporization, or extinction of the first wire 3 and the second wire 4 disposed in the vicinity of the irradiation region of the laser light. Further, the irradiation conditions of the laser light at step S13 are the same as the irradiation conditions of the laser light at step S25. In other words, since it is possible to make the irradiation conditions of the laser light the same in differing processes, a manufacturing operation of the catheter 1 can be made efficient.

At the bonding intersection point Qc[x], the first wire 3[x] is disposed to the outer side in the third direction with respect to the second wire 4[x]. At the bonding intersection point Q1[x] at which the first wire 3[x + 1], which is adjacent to the first wire 3[x], intersects the second wire 4[x], the first wire 3[x + 1] is disposed to the outer side in the third direction with respect to the second wire 4[x]. In this case, when irradiating the laser light at step S13 and bonding the first wire 3[x] and the second wire 4[x] at the bonding intersection point Qc[x], it is possible to suppress the second wire 4[x] from moving to the outer side in the third direction as a result of the first wire 3[x + 1] pressing the second wire 4[x] from the outer side in the third direction. Further, at the bonding intersection point Q2[x] at which the second wire 4[x + 1], which is adjacent to the second wire 4[x], intersects the first wire 3 [x], the second wire 4[x + 1] is disposed to the outer side in the third direction with respect to the first wire 3[ x ]. In this case, when irradiating the laser light at step S13 and bonding the first wire 3[x] and the second wire 4[x] at the bonding intersection point Qc[x], it is possible to suppress the first wire 3[x] from moving to the outer side in the third direction as a result of the second wire 4[x + 1] pressing the first wire 3[x] from the outer side in the third direction. The respective positions of the first wire 3[x] and the second wire [x] at the time of bonding can therefore be made stable, and thus, the first wire 3[x] and the second wire 4[x] can be bonded in a stable manner by the bonding portion 5[x].

The bonding portions 5[n] and 5[n + 1] are disposed to be aligned in the extension direction. The bonding portions 5[n + 2] and 5[n + 3] are disposed to be aligned in the extension direction. The bonding portions 5[n + 4] and 5[n + 5] are disposed to be aligned in the extension direction. Further, the bonding portions 5[n], 5[n + 2], and 5[n + 4] are aligned in the direction orthogonal to the extension direction. The bonding portions 5[n + 1], 5[n + 3], and 5[n + 5] are aligned in the direction orthogonal to the extension direction. By disposing the bonding portions 5 in this manner, the reinforcement member 2 is cut in the direction orthogonal to the extension direction, and the distal end portion 2D and the proximal end portion 2P can be formed.

At step S17 and step S25, the laser light is irradiated in the state in which the inert gas in the laminar flow state is blown onto the reinforcement member 2. In this case, at the time of heat generation of the first wire 3 and the second wire 4 due to the irradiation of the laser light, surrounding air does not come into contact with the first wire 3 and the second wire 4, and it is thus possible to suppress the occurrence of burning by oil and oxidation of the first wire 3 and the second wire 4 resulting from the laser irradiation.

In the long wire material 10 that is prepared in the preliminary process at step S 11, the inner layer tube 6 is disposed around the metal wire 100, and the metal wire 100 and the inner layer tube 6 are disposed in the lumen 2L of the reinforcement member 2. Further, after completing the cutting of both ends, in the extension direction, of the reinforcement member 2, in the latter process at step S33, the outer layer tube 7 is disposed around the reinforcement member 2. In this way, the catheter 1 can be manufactured that is provided with the reinforcement member 2, the inner layer tube 6, and the outer layer tube 7. In particular, by using the metal wire 100, the catheter 1 provided with the thin inner layer tube 6 can be easily manufactured. Further, the surface of the metal wire 100 is plated with the silver color, and reflects the laser light irradiated in the manufacturing process. In this case, it is possible to suppress the irradiated laser light from passing through the lumen 6L of the inner layer tube 6 and reaching the opposite side. As a result, it is possible to reduce the possibility of the reinforcement member 2 on the opposite side being melted by the laser light.

The light transmittance of the inner layer tube 6 is higher than the light transmittance of the outer layer tube 7, and it is thus possible to suppress the inner layer tube 6 from generating heat due to absorbing the laser light. Thus, it is possible to reduce the possibility that the inner layer tube 6 may melt or be damaged due to heat generation resulting from the irradiation of the laser light.

The thermal conductivity of the metal wire 100 is set to be from 350 to 450 w/mK. In the present embodiment, by using the material having the high thermal conductivity as the material of the metal wire 100, thermal diffusion is boosted. In this way, by using the material having the high thermal conductivity in the metal wire 100 also, it is possible to suppress a local temperature increase of the metal wire 100 due to the thermal diffusion. It is thus possible to reduce the possibility of the inner layer tube 6 melting as a result of the temperature increase of the metal wire 100.

### Modified Examples

The present disclosure is not limited to the above-described embodiment, and various modifications are possible. The bonding portion 5[x] that bonds the first wire 3[x] and the second wire 4[x] may be formed only at the distal end side of the reinforcement member 2, and need not necessarily be formed at the proximal end side. Further, the bonding portion 5[x] that bonds the first wire 3 [x] and the second wire 4[x] may be formed only at the proximal end side of the reinforcement member 2, and need not necessarily be formed at the distal end side. The difference in the melting point between the plurality of first wires 30 and the plurality of second wires 40 is approximately 2000°C. In contrast to this, the melting point of the plurality of first wires 30 may be higher than the melting point of the plurality of second wires 40 by approximately 500°C or more. In this case, copper may be used as the material of the plurality of first wires 30, and SUS may be used as the material of the plurality of second wires 40. Further, the melting point of the plurality of first wires 30 may be higher than the melting point of the plurality of second wires 40 by approximately 700°C or more. in this case, aluminum may be used as the material of the plurality of first wires 30, and SUS may be used as the material of the plurality of second wires 40. Further, the melting point of the plurality of first wires 30 may be higher than the melting point of the plurality of second wires 40 by approximately 1000°C or more. In this case, aluminum may be used as the material of the plurality of first wires 30, and titanium may be used as the material of the plurality of second wires 40. In the above-described embodiment, the melting point of the plurality of first wires 30 is set to be higher than the melting point of the plurality of second wires 40 by 500°C or more, but is preferably set to be higher by 700°C or more, and is more preferably set to be higher by 1000°C or more.

The curved portion 2R of the catheter 1 may be provided in the vicinity of the proximal end portion 2P, or may be provided at both the distal end portion 2D and the proximal end portion 2P. The curved portion 2R of the catheter 1 may be formed by the user. In this case, the catheter 1 may be shipped in a state in which the catheter 1 extends in a straight line in the extension direction. The catheter 1 may be used after the curved portion 2R is formed by the user at the distal end portion 2D

Of the second wire 4[x], the portion further to the second distal end side than the bonding intersection point Qc[x] may extend in a straight line without curving. In the above description, of the first wire 3 [x], the end portion on the first distal end side, that is, the end portion thermally cut by the irradiation of the laser light is separated to the first distal end side in the first direction from the bonding intersection point Qc[x] by 0.03 mm to 0.15 mm, but this distance can be changed as appropriate.

The catheter 1 may include only the inner layer tube 6 and the reinforcement member 2, and need not necessarily be provided with the outer layer tube 7. Further, the catheter 1 may include only the reinforcement member 2 and the outer layer tube 7, and need not necessarily be provided with the inner layer tube 6. The hardness of the outer layer tube 7 may be uniform across the whole area thereof in the extension direction. The inner layer tube 6 may be provided with portions for which the hardness differs. Each of the portions at which the hardness of the inner layer tube 6 differs may be arrayed in the extension direction. The thickness of the outer layer tube 7 need not necessarily be uniform in the extension direction, and a plurality of portions having differing thicknesses may be arrayed in the extension direction. The catheter 1 may be configured by the reinforcement member 2 only.

The light transmittance of the inner layer tube 6 is not limited to that of the above-described embodiment, and may be another value. The light transmittance of the inner layer tube 6 may be the same as the light transmittance of the outer layer tube 7, or may be lower than the light transmittance of the outer layer tube 7. The material of the inner layer tube 6 is not limited to PTFE, and may be another material. The absorption coefficient of the plurality of first wires 30 may be the same as the absorption coefficient of the plurality of second wires 40, or may be smaller than the absorption coefficient of the plurality of second wires 40.

The cross-sectional shape of the plurality of first wires 30 is not limited to being a perfect circle and may be elliptical. The cross-sectional shape of the plurality of first wires 30 may be rectangular and the cross-sectional shape of the plurality of second wires 40 may be circular. Both the cross-sectional shape of the plurality of first wires 30 and the plurality of second wires 40 may be one of either circular or rectangular.

The material of the plurality of first wires 30 is not limited to being tungsten. The material of the plurality of second wires 40 is not limited to being SUS. A variety of chosen materials may be used as the respective materials of the plurality of first wires 30 and the plurality of second wires 40, as long as they satisfy the condition that the melting point of the plurality of first wires 30 be higher than the melting point of the plurality of second wires 40 by 500°C or more. For example, molybdenum, platinum, or gold may be used as the plurality of first wires 30 and nickel alloy, titanium alloy, or aluminum alloy may be used as the plurality of second wires 40. Further, stainless steel having respectively different compositions may be used as the plurality of first wires 30 and the plurality of second wires 40.

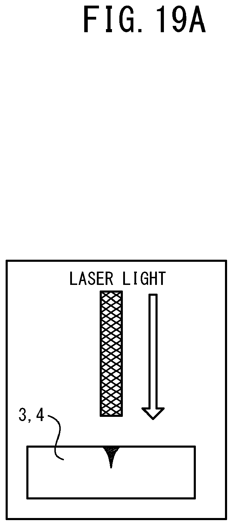

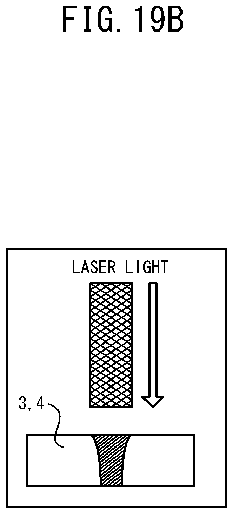

An irradiation pattern when irradiating the laser light at step S13 and step S25 is not limited to that of the above-described embodiment. For example, as shown in Fig. 19A, at step S13 and step S25, the laser light of a relatively low intensity may be first irradiated, and after that, as shown in Fig. 19B, the laser light of a relatively high intensity may be irradiated. Note that, in this case, by first irradiating the laser light having the relatively low intensity, a range of a portion in the first wire 3 and the second wire 4 that generates heat due to the laser light can be restricted. This effect is generally called a slow up effect. Thus, when irradiating the laser light having the relatively high intensity, even if the intensity of the laser light is suppressed, sufficient energy can be imparted to the first wire 3 and the second wire 4. In other words, the energy imparted to the first wire 3 and the second wire 4 can be localized, and it is thus possible to prevent the melting, vaporization, or extinction of the first wire 3 and the second wire 4 in the vicinity of the irradiation region of the laser light.

The output pattern of the laser light at step S13 and step S25 is not limited to that described above. For example, an operation to intermittently irradiate the laser light, and an operation to gradually increase the intensity of the laser light may be combined. The number of times the irradiation of the laser light is intermittently performed is not limited to that of the above-described embodiment. The laser light having the relatively high intensity may be irradiated first, and after that, the laser light having the relatively low intensity may be irradiated. A change in the irradiation mode of the laser light may be performed at step S13 only and need not necessarily be performed at step S25. Conversely, a change in the irradiation mode of the laser light may be performed at step S25 only and need not necessarily be performed at step S13. The laser light irradiated at step S13 and step S25 may be continuously irradiated at a uniform intensity. In other words, the energy imparted to the braided body 20 by the irradiation of the laser light need not necessarily change over time.

The braided body 20 may be a so-called plain weave metal mesh, which is a metal mesh in which the plurality of first wires 30 and the plurality of second wires 40 are caused to be interwoven with each other one wire at a time. Further, the form in which the plurality of first wires 30 and the plurality of second wires 40 are interwoven is not limited to the plain weave metal mesh or the twill weave metal mesh, and may be another form. In this case, at the bonding intersection point Q1[x], the first wire 3[x + 1] may be disposed on the inner side in the third direction with respect to the second wire 4[x]. At the bonding intersection point Q2[x], the second wire 4[x + 1] may be disposed on the inner side in the third direction with respect to the first wire 3[x].

Instead of the blowing of the inert gas in the laminar flow state that is started at step S15 and step S23, the inert gas may be inserted and filled as an atmosphere around the reinforcement member 2 before the laser light is irradiated. The material and conductivity of the metal wire 100 using in the manufacturing process of the catheter 1 are not limited to those of the above-described embodiment. The metal wire 100 need not necessarily be used in the manufacturing process of the catheter 1.

## Claims

1. A catheter comprising:
a cylindrical reinforcement member extending in an extension direction, formed from a braided body obtained by weaving, into a mesh shape, a plurality of first wires extending in a first direction and a plurality of second wires extending in a second direction intersecting the first direction, wherein
a melting point of the plurality of first wires is 500°C or more than a melting point of the plurality of second wires,
at at least one end, of both of end portions in the extension direction of the reinforcement member, bonding portions are formed on some of a plurality of intersection points of intersection between the plurality of first wires and the plurality of second wires, and
the bonding portion
bonds a first intersecting wire and a second intersecting wire, the first intersecting wire and the second intersecting wire being the plurality of first wires and the plurality of second wires intersecting with each other at the some of the plurality of intersection points, and
bonds the first intersecting wire and the second intersecting wire by alloying the first intersecting wire and the second intersecting wire at a contact portion between each of the first intersecting wire and the second intersecting wire.

2. The catheter according to claim 1, wherein
of the plurality of first wires and the plurality of second wires, respectively, at least one end, of both end portions thereof in the extension direction, includes a curved portion curving with respect to the extension direction.

3. The catheter according to claim 1 or 2, wherein
of the second intersecting wire, a second distal end portion further to a distal end side than the bonding portion is wound onto the first intersecting wire.

4. The catheter according to any one of claims 1 to 3, further comprising:
an inner layer tube having a cylindrical shape centered on a central axis of the reinforcement member, and disposed in a lumen of the reinforcement member, and
an outer layer tube having a cylindrical shape centered on the central axis, and covering the reinforcement member from an outer side.

5. The catheter according to claim 4, wherein
a light transmittance of the inner layer tube is higher than a light transmittance of the outer layer tube.

6. The catheter according to claim 4 or 5, wherein
a material of the inner layer tube is polytetrafluoroethylene.

7. The catheter according to any one of claims 1 to 6, wherein
the first intersecting wire is disposed to one side, in a third direction, with respect to the second intersecting wire, the third direction being orthogonal to the first direction and the second direction.

8. The catheter according to any one of claims 1 to 7, wherein
a cross-sectional shape of one of the plurality of first wires and the plurality of second wires is a circular shape, and
the cross-sectional shape of the other of the plurality of first wires and the plurality of second wires is a rectangular shape.

9. The catheter according to claim 8, wherein
the cross-sectional shape of the plurality of first wires is the circular shape, and
the cross-sectional shape of the plurality of second wires is the rectangular shape.

10. The catheter according to claim 9, wherein
an absorption coefficient of the plurality of first wires is greater than an absorption coefficient of the plurality of second wires.

11. The catheter according to any one of claims 1 to 10, wherein
of the second intersecting wire, a composition of a portion other than the bonding portion is the same, regardless of a distance from the bonding portion.

12. The catheter according to any one of claims 1 to 11, wherein
a material of the plurality of first wires is tungsten, and a material of the plurality of second wires is stainless steel.
